Europäisches Patentamt

European Patent Office

Office européen des brevets

(19)

(11) Veröffentlichungsnummer: **0 147 756**
**B1**

(12) # EUROPÄISCHE PATENTSCHRIFT

(45) Veröffentlichungstag der Patentschrift:
**07.10.87**

(51) Int. Cl.⁴: **C 07 C 103/167,** C 07 C 102/08,
C 07 C 103/127

(21) Anmeldenummer: **84115537.7**

(22) Anmeldetag: **15.12.84**

(54) **Verfahren zur Herstellung von beta-Hydroxipropionsäureamid.**

(30) Priorität: **22.12.83 DE 3346415**

(43) Veröffentlichungstag der Anmeldung:
**10.07.85 Patentblatt 85/28**

(45) Bekanntmachung des Hinweises auf die Patenterteilung:
**07.10.87 Patentblatt 87/41**

(84) Benannte Vertragsstaaten:
**AT BE CH DE FR GB IT LI LU NL SE**

(56) Entgegenhaltungen:
**EP - A - 0 078 178**
**DE - B - 2 448 603**
**US - A - 3 670 020**

(73) Patentinhaber: **Chemische Fabrik Stockhausen GmbH,
Bäkerpfad 25, D-4150 Krefeld (DE)**

(72) Erfinder: **Dahmen, Kurt, Dr. Dipl.-Chem., von
Velsen-Strasse 6, D-4050 Mönchengladbach 2 (DE)**
Erfinder: **Reinelt, Peter, Dr. Dipl.-Chem., Grevenbroicher
Strasse 21, D-4150 Krefeld (DE)**
Erfinder: **Brehm, Helmut, Dipl. Ing., Dachstrasse 22,
D-4150 Krefeld (DE)**

(74) Vertreter: **Klöpsch, Gerald, Dr.-Ing., An Gross St.
Martin 6, D-5000 Köln 1 (DE)**

ACTORUM AG

## Beschreibung

Die Erfindung betrifft ein Verfahren zur Herstellung von β-Hydroxypropionsäureamid (β-HPA) durch Umsetzung von Acrylnitril mit Wasser in Gegenwart von als heterogenen Katalysatoren wirksamen Metallen und/oder Metallverbindungen.

β-Hydroxypropionsäureamid wird schon seit längerem in der Literatur beschrieben: Bekannt ist die Reaktion von β-Propiolacton mit Ammoniak zu β-HPA (US-PS 2 375 005), wobei das (krebserzeugende) β-Propiolacton aus Keten und Formaldehyd hergestellt wird.

Weiter kann nach US-PS 2 415 645 Ethylencyanhydrin durch katalytische Verseifung in β-HPA umgewandelt werden. Die Herstellung des Ethylencyanhydrins erfolgt dabei durch Umsetzung von Ethylenoxid mit Cyanwasserstoff.

Eine weitere Möglichkeit zur Herstellung von β-HPA geht von Acrylnitril aus. Wenn Nitrile unter basischen Bedingungen verseift werden, so entstehen gewöhnlich Salze der entsprechenden Carbonsäuren. Bei der Hydrolyse von Acrylnitril mit wässriger alkalischer Lösung findet man normalerweise als Reaktionsprodukte Salze der Acryl- bzw. der β-Hydroxypropionsäure [J. Soc. Chem. Ind. Japan 44, 860 (1941)]. Die Hydrolyse des Acrylnitrils auf der Stufe des Amids zu halten ist nicht einfach, da die Hydrolysegeschwindigkeit der Amide zu den Säuren erheblich höher ist als die Geschwindigkeit der Amidbildung aus dem Nitril.

Beschrieben wird die Hydrolyse von Acrylnitril in Gegenwart von Wasser und heterogenen Katalysatoren zu Acrylamid als Reaktionsprodukt. Als Katalysatoren werden z.B. metallisches Kupfer zusammen mit Kupfer(I)- und/oder Kupfer(II)salzen (US-PS 3 381 034), aktiviertes $MnO_2$ (DE-AS 1 593 320), reduziertes CuO bzw. reduziertes Kupfer-Chromoxyd (DE-OS 2 001 903) oder Raney-Kupfer bzw. andere Metallkatalysatoren (DE-OS 2 036 126) verwendet. Es ist somit zwar bekannt, Acrylamid durch Umsetzung von Acrylnitril mit Wasser in Gegenwart von Metallkatalysatoren wie Raney-Kupfer, Ullmann-Kupfer, anderen Raney-Metallen oder Kupferkatalysatoren, die weitere Metalloxide enthalten, herzustellen (DE-OS 2 036 126), jedoch ist es nicht möglich, diese Hydratationsreaktion, die unter neutralen Bedingungen zum Acrylamid ablaufen muss, auf die Herstellung von β-Hydroxypropionamid anzuwenden.

Eine gezielte Umwandlung von Acrylnitril zu β-HPA wird in US-PS 3 670 020 beschrieben. Als Katalysatoren für die basische Hydrolyse werden Alkali- oder Erdalkalimetallhydroxide sowie organische Substanzen wie tertiäre oder quaternäre Oniumhydroxide und Oniumsalze genannt. Um eine möglichst hohe Ausbeute an β-HPA zu erhalten, wird in dem beschriebenen Verfahren ein Druck von über 20 bar benötigt. Bei Verwendung von NaOH als Katalysator wird mit steigender β-HPA-Ausbeute eine steigende Produktion von β-Hydroxypropionsäure beobachtet. Das Auftreten von β-Hydroxypropionsäure als Nebenprodukt verhindert aber die Kristallisation von β-HPA, so dass eine einfache Aufarbeitung des Reaktionsprodukts behindert ist. Erwünscht wäre daher ein einfaches Verfahren zur Herstellung von β-HPA aus Acrylnitril, das bei relativ niedrigem Druck hohe Ausbeuten an β-HPA ohne Bildung der unerwünschten β-Hydroxypropionsäure liefert.

Überraschenderweise wurde gefunden, dass Acrylnitril bei einer Temperatur höher als Raumtemperatur bis 200°C in Gegenwart von Metallen und/oder Metallverbindungen als Katalysatoren, bevorzugt von Raney-Metallen und/oder deren Metallverbindungen, in einem eng begrenzten pH-Wertbereich, nämlich von 11,8 bis 13,5, mit Wasser, vorzugsweise entsalztem, in hohen Ausbeuten zu β-HPA umgesetzt werden kann, wobei keine oder nur geringe Mengen an β-Hydroxypropionsäure als Nebenprodukt gebildet werden.

Gegenstand der Erfindung ist ein Verfahren zur Herstellung von β-Hydroxypropionsäureamid durch Umsetzung von Acrylnitril mit Wasser in Gegenwart von basischen Katalysatoren, das dadurch gekennzeichnet ist, dass die Reaktion in Gegenwart von Metallen und/oder Metallverbindungen als heterogenen Katalysatoren bei pH-Werten von 11,8 bis 13,5 und Temperaturen zwischen 20°C und 200°C durchgeführt wird.

Bevorzugt wird die Umsetzung bei pH-Werten von 12,8 bis 13,2 durchgeführt. Die Reaktionstemperatur liegt bevorzugt in einem Bereich von 70 bis 200, insbesondere 90 bis 170°C.

Als Katalysatoren werden bevorzugt Raney-Metalle eingesetzt. Das Raney-Metall kann Raney-Kupfer, Raney-Nickel oder Raney-Kobalt sein. Anstelle des Raney-Metalls oder zusätzlich zum Raney-Metall dienen als Katalysatoren Metallverbindungen, und zwar vorzugsweise der vorstehend genannten Raney-Metalle. Als Metallverbindungen kommen die Oxide und Hydroxyde ebenso in Frage wie die Salze der Raney-Metalle. Als geeignete Metallverbindungen seien beispielsweise genannt Kupfer(II)oxid oder Kobalt(III)oxid oder Kupfer(II)salze wie basisches Kupfercarbonat, Kupferoxychlorid, Kupfersulfat oder Kupfernitrat oder Kobaltverbindungen wie $Co(OH)_2$.

Die Reaktion kann sowohl in Gegenwart eines Raney-Metalls oder einer der aufgeführten Metallverbindungen als auch in Gegenwart einer Kombination aus Raney-Metall und Metallverbindung durchgeführt werden. Im letzteren Fall richten sich die Einsatzmengen nach den Mengenverhältnissen, die bei den Einzelkomponenten gelten. Die Reaktionskomponenten sollten vor der Reaktion von Sauerstoff befreit werden, dessen Anwesenheit zur Bildung von unterwünschter β-Hydroxypropionsäure und zur verstärkten Bildung von Metallionen im Produkt beiträgt.

Das Gewichtsverhältnis von Raney-Metall zu Acrylnitril beträgt vorteilhaft 0,01 bis 2, vorzugsweise 0,1 bis 1,0. Das Gewichtsverhältnis von Metallverbindung zu Acrylnitril liegt vorteilhaft bei 1,0 bis 0,0005. Beim erfindungsgemässen Verfahren werden die Reaktionskomponenten in einem Reaktor unter Rühren auf die Reaktionstemperatur (zwischen 20°C und 200°C, bevorzugt höher als 70°C, insbesondere auf 90 bis 170°C) erhitzt. Bei Temperaturen über 100°C wird die Reaktion in einem druckfesten Reaktor durchgeführt, wobei sich ein Druck von 1 bis 10 bar einstellt. Die Umsetzung wird durch Druck nicht besonders beeinflusst.

Das Gewichtsverhältnis von Acrylnitril zu Wasser wird vorteilhaft von 0,01 bis 1,47, vorzugsweise von 0,10 bis 0,74 variiert.

Zur Erhöhung der Hydratationsgeschwindigkeit des Acrylnitrils kann als Aktivator eine Nitratverbindung, z.B. Aluminiumnitrat, zugegeben werden, wobei auch die Gebrauchsdauer des metallischen Katalysators während längerer Zeiten erhalten bleibt. Die Menge an Nitration beträgt vorteilhaft 1 bis 600 ppm, berechnet als Gewichtsverhältnis des Nitrations zum Bruttogewicht der Mischung aus Acrylnitril und Wasser.

Die Einstellung des gewünschten pH-Werts erfolgt durch Zugabe von Basen.

Als Basen können Alkalimetallhydroxide, wie z.B. Lithiumhydroxid, Natriumhydroxid oder Kaliumhydroxid, oder quaternäre Ammoniumhydroxide, wie z.B. Trimethylbenzylammoniumhydroxid, verwendet werden. Die Menge an Base wird so gewählt, dass der pH-Wert der Lösung zwischen 11,8 und 13,5, vorzugsweise zwischen 12,8 und 13,2 beträgt.

Nach einer Reaktionszeit von 5 Min. bis 24 Std. kann das Produkt aufgearbeitet werden. Die Aufarbeitung kann die Stufen Filtration, Kationenaustausch und Anionenaustausch umfassen, eine anschliessende destillative Entfernung der flüchtigen Bestandteile sollte unter vermindertem Druck bei Temperaturen unter 130°C erfolgen.

Aus dem so erhaltenen wasserfreien Produkt kann das $\beta$-Hydroxypropionamid durch Kristallisation gewonnen werden, wobei eine Temperatur von kleiner 0°C von Vorteil ist. Durch Dekristallisation aus kurzkettigen aliphatischen Ketonen wie z.B. Azeton oder Methylethylketon kann das Produkt noch gereinigt werden.

Der nach dem erfindungsgemässen Verfahren eingesetzte Katalysator, bestehend aus Raney-Metall und Metalloxid, kann mit Erfolg mehrfach wiederverwendet werden, ohne dass eine nennenswerte Abnahme der Aktivität des Katalysators eintritt.

Das erfindungsgemäss hergestellte $\beta$-HPA kann als Ausgangsprodukt für die Herstellung von $\alpha,\beta$-ungesättigten substituierten Carbonsäureamiden gemäss US-PS 4 237 067 oder US-PS 4 408 073 verwendet werden, die ihrerseits wieder zu hochmolekularen Flockungsmitteln polymerisiert werden können.

Weiterhin kann es als Ausgangsprodukt zur Herstellung von Esteramiden und Polyesteramiden und als Zwischenprodukt zur Herstellung von Pharmazeutika, Insektiziden und Pestiziden dienen. Auch als Wärmespeichermedium für Wärmepumpen kann es eingesetzt werden.

*Beispiel 1*

In einem druckfesten 2-l-Reaktor aus Edelstahl mit Manometer, Ölbadheizung und Rührwerk wurden 500 g entsalztes Wasser, 2 g NaOH, 75 mg $Al(NO_3)_3 \cdot 9 H_2O$, 100 mg Hydrochinon sowie 70 g Acrylnitril gegeben und mit Stickstoffgas vom gelösten Sauerstoff befreit. 25 g Raney-Kupfer wurden mit sauerstofffreiem Wasser gewaschen und zusammen mit 6 g Kupferoxid-Pulver in den Reaktor gegeben. Der pH-Wert des Gemisches betrug 12,9. Das Gemisch wurde auf 150°C erhitzt, wobei sich ein Druck von ca. 6 bar einstellte. Die Reaktionszeit betrug 3 Stunden. Als Reaktionsprodukt wurde eine klare, grüne Flüssigkeit erhalten. Das Rohprodukt wurde zur Entfernung der Kupferionen über einen Kationenaustauscher gegeben und anschliessend im Vakuum bei 100°C und 0,5 mbar von flüchtigen Bestandteilen befreit. Das zähflüssige, braune Produkt wurde mittels HPLC analysiert; es bestand zu 72 Gew.-% aus $\beta$-Hydroxypropionamid und zu 24 Gew.-% aus Ethylencyanhydrin.

*Beispiel 2*

Es wurde wie in Beispiel 1 vorgegangen, die Menge an Acrylnitril betrug hier 96 g. Der pH-Wert des Gemisches betrug 12,8. Die Reaktionszeit betrug 4 Stunden bei einer Temperatur von 150°C und einem Druck von ca. 6 bar. Als Reaktionsprodukt wurde eine hellgrüne, klare Flüssigkeit erhalten. Das Rohprodukt wurde nach Entfernung der Kupferionen mittels Kationenaustauscher bei 100°C und 0,5 mbar von flüchtigen Bestandteilen befreit. Die Analyse des zähflüssigen, braunen Produkts ergab 70 Gew.-% $\beta$-Hydroxypropionamid und 28 Gew.-% Ethylencyanhydrin.

*Beispiele 3 bis 6*

In diesen Versuchen wurde das Katalysatorsystem, bestehend aus Raney-Kupfer und Kupferoxid, aus Beispiel 2 nochmals benutzt. In den Reaktor mit dem darin befindlichen Raney-Kupfer und dem Kupferoxid wurden 500 g $H_2O$, 2 g NaOH, 100 mg $Al(NO_3)_3 \cdot 9 H_2O$, 100 mg Hydrochinon sowie 70 g Acrylnitril unter Sauerstoffausschluss gegeben. Die pH-Werte der Lösungen betrugen 12,9 bis 13,0. Bei 150°C betrug der Druck ca. 6 bar und die Reaktionszeiten 6 Stunden. Es wurden hellgrüne Flüssigkeiten erhalten. Die Rohprodukte wurden mit einem Kationenaustauscher behandelt und bei 100°C und 0,5 mbar von flüchtigen Bestandteilen befreit.

In der folgenden Tabelle sind die Analysenergebnisse aufgetragen:

| Versuch | % $HOCH_2CH_2CONH_2$ | % $HOCH_2CH_2CN$ | $HOCH_2CH_2COOH$ |
|---|---|---|---|
| 3 | 70 | 28 | — |
| 4 | 54 | 22 | — |
| 5 | 51 | 21 | — |
| 6 | 58 | 37 | Spur |

*Beispiele 7 bis 12*

In diesen Versuchen wurde das Katalysatorsystem variiert, ansonsten wurde wie in Beispiel 1 vorgegangen. Anstelle von NaOH wurde KOH und LiOH eingesetzt, anstelle von Raney-Kupfer wurden Raney-Kobalt bzw. Raney-Nickel und anstelle von CuO wurde $Co_2O_3$ eingesetzt. Die pH-Werte der Mischungen lagen zwischen 12,4 und 13,1. Die Ergebnisse sind in der folgenden Tabelle 1 aufgeführt:

TABELLE 1

| Versuch | Katalysator | % $HOCH_2CH_2CONH_2$ | % $HOCH_2CH_2CN$ | $HOCH_2CH_2COOH$ |
|---|---|---|---|---|
| 7 | Raney-Cu/CuO/NaOH | 65,5 | 31,5 | — |
| 8 | Raney-Cu/CuO/KOH | 47,4 | ca. 30 | — |
| 9 | Raney-Cu/CuO/LiOH | 44,2 | 10,8 | — |
| 10 | Raney-Co/CuO/NaOH | 52,8 | 3,9 | wenig |
| 11 | Raney-Co/$CO_2O_3$/NaOH | 48,4 | ca. 20 | — |
| 12 | Raney-Ni/CuO/NaOH | 53 | ca. 10 | |

*Beispiel 13*

Wie in Beispiel 1 wurden 810 g entsalztes Wasser, 0,5 g NaOH, 100 mg $Al(NO_3)_3 \cdot 9\,H_2O$, 100 mg Hydrochinon und 159 g Acrylnitril in Gegenwart von 80 g Raney-Kupfer zusammengegeben. Das Gemisch hatte einen pH-Wert von 12,0. Nach einer Reaktionszeit von 4 Stunden bei einer Temperatur von 150°C und einem Druck von ca. 6 bar wurde eine klare braune Flüssigkeit erhalten. Das Rohprodukt wurde im Vakuum bei 100°C und 0,5 mbar von flüchtigen Bestandteilen befreit. Das zähflüssige, braune Produkt bestand zu 39,4% aus β-Hydroxypropionamid und zu 17,1% aus Ethylencyanhydrin.

*Beispiel 14*

Es wurden wie in Beispiel 1    432 g entsalztes Wasser, 5 g NaOH, 500 mg $Al(NO_3)_3 \cdot 9\,H_2O$, 100 mg Hydrochinon und 159 g Acrylnitril mit 80 g Raney-Kupfer zusammengegeben. Das Gemisch hatte einen pH-Wert von 13,4. Nach 4 Stunden Reaktionszeit bei einer Temperatur von 150°C und einem Druck von ca. 7 bar wurde eine braune Flüssigkeit erhalten. Nach üblicher Aufarbeitung wurde das Produkt analysiert. Es enthielt 46,5% β-Hydroxypropionamid und 9,7% Ethylencyanhydrin.

*Beispiele 15 bis 22*

Gemäss dem in Beispiel 1 beschriebenen Verfahren wurden in den Versuchen 15 bis 22 jeweils 810 g entsalztes Wasser, 100 mg $Al(NO_3)_3 \cdot 9\,H_2O$, 100 mg Hydrochinon, 159 g Acrylnitril sowie die in Tabelle 2 angegebenen Mengen an Natriumhydroxid und an Metallverbindungen zur Reaktion gebracht. Nach üblicher Aufarbeitung wurden in den Produkten die Gehalte an β-HPA ermittelt, die in Tabelle 2 angegeben sind.

TABELLE 2

| Versuch | Katalysator | Menge (g) | NaOH (g) | pH-Wert | % β-HPA | % ECH |
|---|---|---|---|---|---|---|
| 15 | $Cu(NO_3)_2 \cdot 3H_2O$ | 20 | 8 | 12,4 | 28 | 17 |
| 16 | $CuCl_2 \cdot 3Cu(OH)_2$ | 20 | 8 | 13,1 | 39 | 24 |
| 17 | $Cu(OH)_2 \cdot CuCO_3$ | 40 | 5 | 12,8 | 50 | 4 |
| 18 | $CuSO_4 \cdot 6H_2O$ | 20 | 7 | 12,2 | 41 | 21 |
| 19 | $CuSO_4 \cdot 5H_2O$ | 20 | 9 | 12,7 | 32 | 20 |
| 20 | CuO | 20 | 5 | 12,8 | 46 | 5 |
| 21 | CuO | 60 | 5 | 12,8 | 53 | 16 |
| 22 | $Co(OH)_2$ | 20 | 5 | 13,0 | 42 | 41 |

*Beispiel 23*

Gemäss dem in Beispiel 1 beschriebenen Verfahren wurden 810 g entsalztes Wasser, 100 mg $Al(NO_3)_3 \cdot 9\,H_2O$, 100 mg Hydrochinon, 159 g Acrylnitril, 5 g Natriumhydroxid sowie 80 g Raney-Kupfer, das mit Wasser gewaschen war, zusammengegeben. Die Reaktionszeit betrug 4 Stunden bei einer Temperatur von 150°C und einem Druck von 40 bar. Nach üblicher Aufarbeitung ergab die Analyse des Produkts 63% β-HPA und 18% ECH.

*Beispiel 24*

Gemäss dem in Beispiel 1 beschriebenen Verfahren wurden 360 g entsalztes Wasser, 80 mg

Al(NO$_3$)$_3$ · 9 H$_2$O, 100 mg Hydrochinon, 265 g Acrylnitril, 1 g Natriumhydroxid sowie 50 g mit Wasser gewaschenes Raney-Kupfer und 0,4 g Kupferoxid zusammengegeben. Das Gemisch hatte einen pH-Wert von 12,4. Die Reaktionszeit betrug 6 Stunden bei einer Temperatur von 150°C und einem Druck von ca. 6 bar. Nach üblicher Aufarbeitung ergab die Analyse des Produkts 22% β-HPA und 24% ECH.

*Beispiel 25*

Gemäss dem in Beispiel 1 beschriebenen Verfahren wurden 500 g entsalztes Wasser, 100 mg Al(NO$_3$)$_3$ · 9 H$_2$O, 100 mg Hydrochinon, 70 g Acrylnitril, 50 g Raney-Kupfer sowie 5 g Kupferoxid zusammengegeben. Die Einstellung des pH-Werts auf pH 12,4 erfolgt durch Zugabe von 8 g Benzyltrimethylammoniumhydroxid (40%ige Lösung in Wasser). Das Gemisch wurde bei 150°C und einem Druck von ca. 6 bar für 5 Stunden zur Reaktion gebracht. Nach üblicher Aufarbeitung des Produkts ergab die Analyse 48% β-HPA und 20% ECH.

*Beispiel 26*

Gemäss dem in Beispiel 1 beschriebenen Verfahren wurden 500 g entsalztes Wasser, 100 mg Hydrochinon, 300 mg Al(NO$_3$)$_3$ · 9 H$_2$O, 100 g Acrylnitril, 1,5 g NaOH sowie 100 g mit Wasser gewaschenes Raney-Kupfer zur Reaktion gebracht. Die Reaktionstemperatur von 170°C, bei der sich ein Druck von ca. 9 bar einstellte, wurde für die Zeit von 15 Minuten gehalten. Nach üblicher Aufarbeitung ergab die Analyse 56% β-HPA und 11% ECH.

*Beispiel 27*

In einem offenen Glasgefäss mit Rückflusskühler und Rührer wurden 500 g entsalztes Wasser, 300 mg Al(NO$_3$)$_3$ · 9 H$_2$O, 100 mg Hydrochinon, 1,5 g NaOH, 100 g mit Wasser gewaschenes Raney-Kupfer, sowie 100 g Acrylnitril zusammengegeben. Das Gemisch wurde unter ständigem Rühren bei einer Temperatur von 90°C für 3 Stunden gehalten. Nach üblicher Aufarbeitung ergab die Analyse 35% β-HPA und 20% ECH.

*Beispiel 28*

Gemäss dem in Beispiel 1 beschriebenen Verfahren wurden 960 g entsalztes Wasser, 100 mg Hydrochinon, 100 g Acrylnitril, 3 g NaOH sowie 100 g mit Wasser gewaschenes Raney-Kupfer zusammengegeben. Das Gemisch hatte einen pH-Wert von 12,8. Die Reaktionszeit betrug 3 Stunden bei einer Temperatur von 140°C und einem Druck von ca. 5 bar. Nach üblicher Aufarbeitung ergab die Analyse des Produkts 72% β-HPA und 11% ECH.

*Beispiel 29*

Gemäss dem in Beispiel 1 beschriebenen Verfahren wurden 810 g entsalztes Wasser, 100 mg Al(NO$_3$)$_3$ · 9 H$_2$O, 100 mg Hydrochinon, 159 g Acrylnitril, 3 g NaOH sowie 80 g mit Wasser gewaschenes Raney-Kupfer und 0,1 g Kupferoxid zusammengegeben. Das Gemisch hatte einen pH-Wert von 12,8. Die Reaktionszeit betrug 24 Stunden bei einer Temperatur von 150°C und einem Druck von ca. 6 bar. Nach üblicher Aufarbeitung ergab die Analyse des Produkts 57% β-HPA und 29% ECH.

*Vergleichsbeispiel (entsprechend US-PS 3 670 020)*

In diesem Beispiel wurden kein Raney-Metall und kein Metalloxid eingesetzt. Wie in Beispiel 1 wurden bei 150°C während 6 Stunden bei 40 bar ein Gemisch aus 810 g H$_2$O, 14 g NaOH, 100 mg Hydrochinon und 159,2 g Acrylnitril zur Reaktion gebracht. Das Gemisch hatte einen pH-Wert von 13,5. Nach üblicher Aufarbeitung ergab die Analyse des Produkts: 27,4% β-Hydroxypropionamid, 12,1% Ethylencyanhydrin, ca. 35% Hydroxypropionsäure.

*Vergleichsbeispiel (entsprechend DE-OS 2 036 126)*

In diesem Beispiel wurde wie in Beispiel 1 vorgegangen, jedoch kein NaOH zugesetzt. Das Gemisch hatte einen pH-Wert von 9,6. Die Analyse des wie üblich aufgearbeiteten Produkts ergab: 10,5% β-Hydroxypropionamid, 4,3% Ethylencyanhydrin, Hauptkomponente Acrylamid.

**Patentansprüche**

1. Verfahren zur Herstellung von β-Hydroxypropionsäureamid durch Umsetzung von Acrylnitril mit Wasser in Gegenwart von basischen Katalysatoren, dadurch gekennzeichnet, dass man die Umsetzung in Gegenwart von Metallen und/oder Metallverbindungen als heterogenen Katalysatoren bei pH-Werten von 11,8 bis 13,5 und Temperaturen zwischen 20°C und 200°C durchführt.

2. Verfahren nach Anspruch 1, dadurch gekennzeichnet, dass man die Umsetzung bei pH-Werten von 12,8 bis 13,2 durchführt.

3. Verfahren nach Anspruch 1 oder 2, dadurch gekennzeichnet, dass man die Umsetzung bei Temperaturen von 70 bis 200, vorzugsweise bei 90 bis 170°C durchführt.

4. Verfahren nach Ansprüchen 1 bis 3, dadurch gekennzeichnet, dass als Katalysatoren Raney-Metalle und/oder Metallverbindungen dieser Raney-Metalle, vorzugsweise die Oxide, Hydroxide oder Salze verwendet werden.

5. Verfahren nach Ansprüchen 1 bis 4, dadurch gekennzeichnet, dass man ein Gewichtsverhältnis von Raney-Metall zu Acrylnitril von 0,01 bis 2, vorzugsweise 0,1 bis 1,0 einsetzt.

6. Verfahren nach Anspruch 5, dadurch gekennzeichnet, dass das Gewichtsverhältnis von Metallverbindung zu Acrylnitril 0,0005 bis 1,0 beträgt.

7. Verfahren nach Ansprüchen 1 bis 6, dadurch gekennzeichnet, dass man die Umsetzung mit Gewichtsverhältnissen von Acrylnitril zu Wasser von 0,01 bis 1,47, vorzugsweise 0,10 bis 0,74 durchführt.

8. Verfahren nach Ansprüchen 1 bis 7, dadurch gekennzeichnet, dass man die Umsetzung in Gegenwart einer Nitratverbindung, vorzugsweise Aluminiumnitrat durchführt.

9. Verfahren nach Anspruch 8, dadurch gekennzeichnet, dass man die Nitratverbindung in einer Menge von 1 bis 600 ppm einsetzt, berechnet als Gewichtsverhältnis des Nitrat-Ions zum Bruttogewicht der Mischung aus Acrylnitril und Wasser.

## Claims

1. A process for the production of beta-hydroxypropionic acid amide by the conversion of acrylonitrile with water in the presence of basic catalysts, characterized in that the conversionis carried out in the presence of metals and/or metallic compounds as heterogenous catalysts at pH values from 11.8 to 13.5, and at temperatures between 20°C and 200°C.

2. A process according to claim 1, characterized in that the conversion is carried out at pH values from 12.8 to 13.2.

3. A process according to claim 1 or 2, characterized in that the conversion is carried out at temperatures of 70°C to 200°C, preferably at 90°C to 170°C.

4. A process according to any one of claims 1 to 3, characterized in that Raney metals and/or metallic compounds of these Raney metals, preferably the oxides, hydroxides or salts of these Raney metals, are used as catalysts.

5. A process according to any one of claims 1 to 4, characterized in that a weight ratio of Raney metal to acrylonitrile of 0.01 to 2, preferably of 0.1 to 1.0, is used.

6. A process according to claim 5, characterized in that the weight ratio of metallic compound to acrylonitrile amounts from 0.0005 to 1.0.

7. A process according to any one of claims 1 to 6, characterized in that the conversion is carried out at weight ratios of acrylonitrile to water of 0.01 to 1.47, preferably of 0.10 to 0.74.

8. A process according to any one of claims 1 to 7, characterized in that the conversion is carried out in the presence of a nitrate compound, preferably in the presence of aluminum nitrate.

9. A process according to claim 8, characterized in that the nitrate compound is used in a quantity of 1 to 600 ppm, calculated as a weight ratio of the nitrate ion to the gross weight of the mixture of acrylonitrile and water.

## Revendications

1. Procédé de préparation de l'amide de l'acide β-hydroxy-propionique par réaction du nitrile acrylique et d'eau en présence de catalyseurs basiques, caractérisé en ce que la réaction est réalisée entre 20 et 200°C à un pH compris entre 11,8 et 13,5 en présence de métaux et(ou) de dérivés de métaux comme catalyseurs hétérogènes.

2. Procédé suivant la revendication 1, caractérisé en ce que la réaction est effectuée à un pH compris entre 12,8 et 13,2.

3. Procédé suivant la revendication 1 ou 2, caractérisé en ce que la réaction est effectuée à des températures de 70 à 200 et de préférence de 90 à 170°C.

4. Procédé suivant les revendications 1 à 3, caractérisé en ce que les catalyseurs utilisés sont des métaux de Raney et(ou) des dérivés de métaux de Raney, en particulier des oxydes, hydroxydes ou sels.

5. Procédé suivant les revendications 1 à 4, caractérisé en ce que le rapport pondéral du métal de Raney au nitrile acrylique est compris entre 0,01 et 2 et de préférence entre 0,1 et 1,0.

6. Procédé suivant la revendication 5, caractérisé en ce que le rapport pondéral entre le dérivé de métal et le nitrile acrylique est compris entre 0,0005 et 1,0.

7. Procédé suivant les revendications 1 à 6, caractérisé en ce que la réaction est réalisée avec des proportions pondérales de nitrile acrylique et d'eau dans un rapport de 0,01 à 1,47 et de préférence de 0,10 à 0,74.

8. Procédé suivant les revendications 1 à 7, caractérisé en ce que la réaction est réalisée en présence d'un nitrate, de préférence du nitrate d'aluminium.

9. Procédé suivant la revendication 8, caractérisé en ce que le nitrate est employé en une proportion de 1 à 600 ppm d'ions nitrate par rapport au poids total brut du mélange du nitrile acrylique et de l'eau.